# EUROPÄISCHE PATENTANMELDUNG

(11) **EP 1 820 783 A1**
(43) Veröffentlichungstag der Anmeldung: **22.08.2007**
(21) Anmeldenummer: 07450030.7
(22) Anmeldetag: 19.02.2007
(51) Int. Cl.: C02F 11/04, C02F 3/28, C12M 1/107, B65D 88/60

(54) **Vorrichtung zur Gärgutbeschickung einer Gäranlage**

(30) Priorität: 21.02.2006 AT 2792006
(71) Anmelder: MARATON Energie Technik GmbH, 5211 Friedburg (AT)
(72) Erfinder: Metzger, Reinhard, A-5310 Mondsee (AT)
(74) Vertreter: Hübscher, Helmut

(57) **Zusammenfassung**

Es wird eine Vorrichtung zur Gärgutbeschickung einer Gäranlage mit einer das Gärgut zwischen zwei einander gegenüberliegenden Längswänden (3) aufnehmenden Vorratskammer (1) und mit einem zwischen den Längswänden (3) entlang einer Längsführung schrittweise gegen eine stirnseitige Austragsöffnung der Vorratskammer (1) vorschiebbaren Schubschild (4) beschrieben. Um vorteilhafte Vorschubbedingungen zu schaffen, wird vorgeschlagen, daß die Längsführung für das Schubschild (4) entlang der Längswände (3) verlaufende Führungsschienen (11) für seitliche Führungswagen (9) aufweist, die das Schubschild (4) in Richtung der Längsführung verschiebbar tragen, und daß zwischen den Führungswagen (9), die abwechselnd mit dem Schubschild (4) entlang der Führungsschienen (11) im Abstand aufeinanderfolgender Förderschritte verriegelbar sind, und dem Schubschild (4) ein Vorschubantrieb (20) vorgesehen ist.

## Beschreibung

Die Erfindung bezieht sich auf eine Vorrichtung zur Gärgutbeschickung einer Gäranlage mit einer das Gärgut zwischen zwei einander gegenüberliegenden Längswänden aufnehmenden Vorratskammer und mit einem zwischen den Längswänden entlang einer Längsführung schrittweise gegen eine stirnseitige Austragsöffnung der Vorratskammer vorschiebbaren Schubschild.

Zur Beschickung von Gäranlagen für eine Biogaserzeugung wird das jeweils benötigte Gärgut, üblicherweise nachwachsende Rohstoffe, von einem Gutstock abgetragen und einem weiterführenden Förderer aufgegeben. Zu diesem Zweck ist es bekannt, das Gärgut in eine zwischen zwei Längswänden gebildete Vorratskammer einzubringen, die an ihrem stirnseitigen Entnahmeende eine über die Gutstockhöhe verfahrbare Abtragseinrichtung, üblicherweise eine Walzenfräse, aufweist. Der Gutstock wird dabei durch ein zwischen den beiden Längswänden vorgesehenes Schubschild schrittweise gegen die Abtragseinrichtung vorgeschoben, so daß die Länge der Förderschritte das in einem Arbeitsgang vom Gutstock abfräsbare Gutvolumen bestimmt. Nachteilig bei bekannten Beschickungsvorrichtungen dieser Art ist vor allem, daß die für den Vorschubantrieb des Schubschildes vorgesehenen Vorschubzylinder aufgrund des durch die Länge der Vorratskammer vorgegebenen Stellweges entsprechend goß und schwer ausgebildet werden müssen, wozu noch kommt, daß diese Vorschubzylinder schrittweise aufeinanderfolgende Hübe ausführen müssen, was eine vergleichsweise aufwendige Steuerung der Vorschubzylinder bedingt. Schließlich ergeben sich hinsichtlich der Beladung der Vorratskammer mit Gärgut Schwierigkeiten, weil das Gärgut nur von oben in die Vorratskammer eingebracht werden kann.

Der Erfindung liegt somit die Aufgabe zugrunde, eine Vorrichtung zur Gärgutbeschickung einer Gäranlage der eingangs geschilderten Art so auszugestalten, daß eine kompakte Bauweise mit einem wenig ausladenden, einfach anzusteuernden Vorschubantrieb für das Schubschild erhalten wird. Darüber hinaus sollen Konstruktionsvoraussetzungen geschaffen werden, um im Bedarfsfall die Vorratskammer vorteilhaft mit Gärgut beladen zu können.

Die Erfindung löst die gestellte Aufgabe dadurch, daß die Längsführung für das Schubschild entlang der Längswände verlaufende Führungsschienen für seitliche Führungswagen aufweist, die das Schubschild in Richtung der Längsführung verschiebbar tragen, und daß zwischen den Führungswagen, die abwechselnd mit dem Schubschild entlang der Führungsschienen im Abstand aufeinanderfolgender Förderschritte verriegelbar sind, und dem Schubschild ein Vorschubantrieb vorgesehen ist.

Da das Schubschild gegenüber den es tragenden Führungswagen in Richtung der Längsführung verschiebbar gelagert ist und unabhängig von den Führungswagen wie diese mit den Führungsschienen verriegelt werden kann, sind vorteilhafte Voraussetzungen für einen schrittweisen Schildvorschub mit Hilfe eines Vorschubantriebes geschaffen, dessen maximaler Hub auf die größte Länge eines Förderschrittes beschränkt ist. Zum Schildvorschub um einen Förderschritt braucht ja lediglich der Vorschubantrieb bei verriegelten Führungswagen betätigt zu werden. Danach wird das Schubschild gegenüber den Führungsschienen verriegelt, so daß nach einer Entriegelung der Führungswagen diese durch den Vorschubantrieb um einen Förderschritt nachgezogen werden können, um nach einer Verriegelung der Führungswagen und einer Entriegelung des Schubschildes die Ausgangslage für einen weiteren Förderschritt des Schubschildes zu erhalten. Die vergleichsweise kurzen Hübe des Vorschubantriebes erlauben eine wenig ausladende Bauart mit dem Vorteil, daß aufwendige Steuerungsmaßnahmen entfallen können, weil bloß für die abwechselnde Ver- und Entriegelung des Schubschildes und der Führungswagen sowie für eine entsprechende Beaufschlagung des Vorschubantriebes zu sorgen ist. Werden für die Verriegelung der Führungswagen und des Schubschildes Stellzylinder und für den Vorschub Vorschubzylinder eingesetzt, so ergeben sich besonders einfache Konstruktionsverhältnisse, obwohl auch andere Stelltriebe bzw. ein anderer Vorschubantrieb verwendet werden können.

Das Vorsehen von seitlichen, den Führungsschienen entlang verfahrbaren Führungswagen, die das Schubschild verschiebbar tragen, stellt darüber hinaus eine vorteilhafte Voraussetzung für eine Beladung der Vorratskammer mit Gärgut dar. Zu diesem Zweck braucht nämlich das Schubschild nur zwischen auf den Führungswagen gelagerten Längsschlitten um eine Querachse schwenkbar gelagert zu werden, so daß die Vorratskammer entweder unter dem hochgeschwenkten oder über das gegen den Boden der Vorratskammer abgeschwenkten Schubschild beladen werden kann. Besonders vorteilhafte Konstruktionsverhältnisse ergeben sich in diesem Zusammenhang, wenn das Schubschild um eine bodennahe Querachse abschwenkbar zwischen den Längsschlitten gelagert ist und eine befahrbare Brücke bildet. In diesem Fall kann das Gärgut nämlich mittels eines beispielsweise als Ladewagen ausgebildeten Fahrzeuges in die Vorratskammer eingebracht werden, und zwar ohne Höhenbeschränkung durch eine obere Schwenkachse für das Schubschild.

Die Verriegelung der Führungswagen und der in diesen verschiebbar gehaltenen Längsschlitten, zwischen denen das Schubschild gelagert ist, kann vorteilhaft durch Verriegelungsbolzen vorgenommen werden, die an Stelltrieben angeschlossen sind und in im Abstand der Förderschritte vorgesehenen Rastaufnahmen der Führungsschienen eingreifen.

In der Zeichnung ist der Erfindungsgegenstand beispielsweise dargestellt. Es zeigen
Fig. 1 eine erfindungsgemäße Vorrichtung zur Gärgutbeschickung einer Gäranlage in einem vereinfachten Längsschnitt,
Fig. 2 einen Schnitt nach der Linie II-II der Fig. 1 in einem größeren Maßstab und

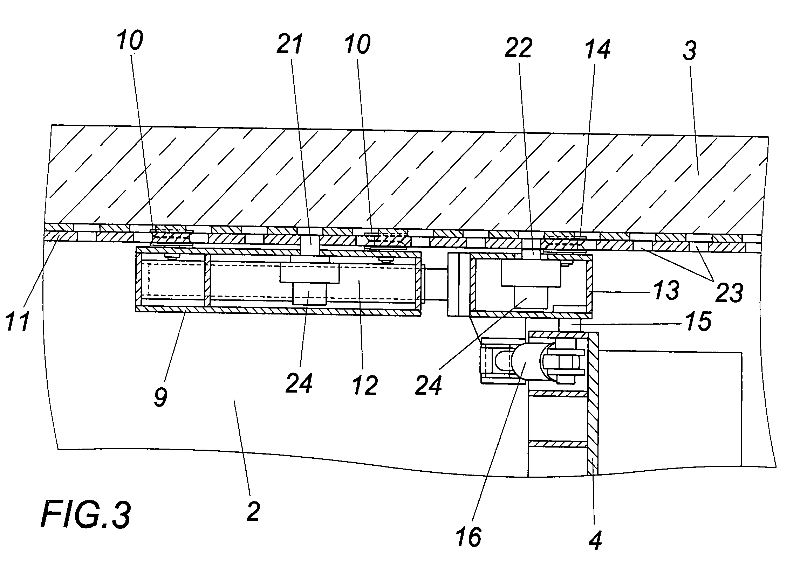

Fig. 3 einen Schnitt nach der Linie III-III der Fig. 2.

Die dargestellte Vorrichtung zur Gärgutbeschickung einer Gäranlage weist eine Vorratskammer 1 zur Aufnahme des Gärgutes auf. Diese Vorratskammer 1 wird durch einen Boden 2 und zwei einander gegenüberliegenden, vom Boden 2 aufragenden Längswänden 3 gebildet, zwischen denen ein Schubschild 4 zum Vorschub des von der Vorratskammer 1 aufgenommenen Gutstockes gegen eine stirnseitige Austragsöffnung der Vorratskammer 1 hin vorgesehen ist. Beim Einsatz von rieselfreudigem Gärgut kann das Gärgut im Ausmaß des Vorschubes durch die stirnseitige Austragsöffnung schwerkraftbedingt einem weiterführenden Förderer aufgegeben werden. Andernfalls ist die austragseitige, offene Stirnseite der Vorratskammer 1 mit einer Abtragseinrichtung 5 abzuschließen, die gemäß dem Ausführungsbeispiel einen über die Höhe des Gutstockes in einem Führungsgestell 6 verlagerbaren Walzenfräser 7 umfaßt, der das vom Gutstock abgefräste Gärgut einem im Boden 2 eingelassenen Querförderer 8 zum Weitertransport aufgibt.

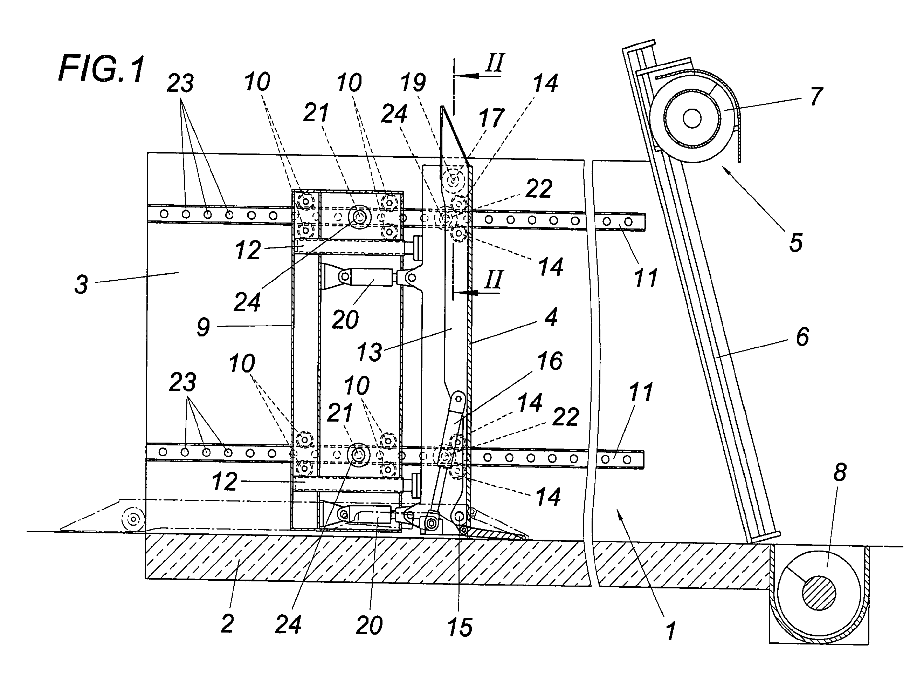

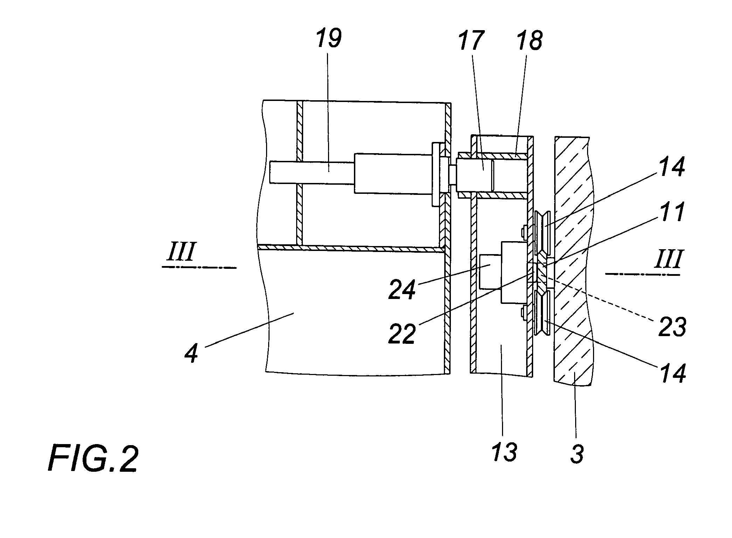

Zur Führung des Schubschildes 4 dienen zwei seitliche Führungswagen 9, die mit Hilfe von Laufrollen 10 auf Führungsschienen 11 der Längswände 3 gelagert sind. Die Führungswagen 9 tragen in Richtung der Führungsschienen 11 verlaufende Gleitführungen 12 für den Führungswagen 9 vorgeordnete Längsschlitten 13, die zusätzlich über Laufrollen 14 auf den Führungsschienen 11 abgestützt sind. Das Schubschild 4 ist zwischen den seitlichen Längsschlitten 13 schwenkbar gelagert, und zwar um eine bodennahe Querachse 15. Zur Schwenkverstellung des Schubschildes 4 zwischen einer in der Fig. 1 in vollen Linien dargestellten, aufrechten Arbeitsstellung und einer gegen den Boden 2 der Vorratskammer 1 abgeschwenkten, strichpunktiert angedeuteten Beladungsstellung dienen Schwenkzylinder 16. Zur Verriegelung des Schubschildes 4 in der aufrechten Arbeitsstellung sind an den Seitenwangen des Schubschildes 4 Riegelbolzen 17 gelagert, die in der Verriegelungsstellung gemäß der Fig. 2 in eine Verriegelungshülse 18 der Längsschlitten 13 eingreifen. Zur Betätigung der Riegelbolzen 17 sind Stellzylinder 19 vorgesehen.

Zwischen den Führungswagen 9 und den Längsschlitten 13 ist ein Vorschubantrieb 20 angeordnet, der paarweise angelenkte Vorschubzylinder umfaßt. Außerdem sind sowohl die Führungswagen 9 als auch die vorgelagerten Längsschlitten 13 mit Verriegelungsbolzen 21, 22 versehen, die mit Rastausnehmungen 23 in den Führungsschienen 11 zusammenwirken. Die Riegelbolzen 21, 22 werden mit Hilfe von Stelltrieben 24 betätigt, deren Ansteuerung so erfolgt, daß die Längsschlitten 13 abwechselnd mit den Führungswagen 9 gegenüber den Führungsschienen 11 verriegelt werden. Dies bedeutet, daß bei einer Verriegelung der Führungswagen 9 die Längsschlitten 13 und damit das Schubschild 4 über den Vorschubantrieb 20 um einen Förderschritt gegen die Abtragseinrichtung 5 vorgeschoben werden kann. Da die Rastausnehmungen 23 in den Führungsschienen 11 einen gegenseitigen Abstand entsprechend der Förderschrittlänge aufweisen, kann am Ende jedes Förderhubes des Vorschubantriebes 20 das Schubschild 4 über die Längsschlitten 13 mit den Führungsschienen 11 verriegelt werden, um die Führungswagen 9 nach deren Entriegelung um einen Förderschritt nachzuziehen, so daß nach der Verriegelung der Führungswagen 9 und der Entriegelung der Längsschlitten 13 die Ausgangslage für einen neuen Förderschritt erreicht wird. Der Gutstock in der Förderkammer 1 kann demnach schrittweise gegen die Abtragseinrichtung 5 vorgeschoben und entsprechend diesen Förderschritten abgetragen werden. Nach einem vollständigen oder weitgehenden Abtrag des Gutstockes wird das Schubschild 4 schrittweise zu der der Abtragseinrichtung 5 gegenüberliegenden Stirnseite der Vorratskammer 1 verlagert, indem wiederum die Führungswagen 9 und die Längsschlitten 13 zwischen den Beaufschlagungen des Vorschubantriebes 20 abwechselnd ver- und entriegelt werden. Wird das Schubschild 4 durch ein Zurückziehen des Verriegelungsbolzens 17 aus der Verriegelungshülse 18 entriegelt, so kann das Schubschild 4 aus der Arbeitsstellung in die in der Fig. 1 strichpunktiert angedeutete Beladungsstellung abgeschwenkt werden, in der das Schubschild 4 eine befahrbare Brücke bildet, über die hinweg die Vorratskammer 1 beispielsweise mit Hilfe eines Ladewagens befahren werden kann, was die Beladung der Vorratskammer 1 mit neuem Gärgut erheblich vereinfacht. Nach der Beladung der Vorratskammer 1 wird das Schubschild 4 wieder in die Arbeitsstellung hochgeschwenkt und verriegelt, so daß die Vorrichtung bei verriegelten Führungswagen 9 und entriegelten Längsschlitten 13 wieder für einen ersten Förderschritt bereitsteht.

## Patentansprüche

1. Vorrichtung zur Gärgutbeschickung einer Gäranlage mit einer das Gärgut zwischen zwei einander gegenüberliegenden Längswänden (3) aufnehmenden Vorratskammer (1) und mit einem zwischen den Längswänden (3) entlang einer Längsführung schrittweise gegen eine stirnseitige Austragsöffnung der Vorratskammer (1) vorschiebbaren Schubschild (4), **dadurch gekennzeichnet, daß** die Längsführung für das Schubschild (4) entlang der Längswände (3) verlaufende Führungsschienen (11) für seitliche Führungswagen (9) aufweist, die das Schubschild (4) in Richtung der Längsführung verschiebbar tragen, und daß zwischen den Führungswagen (9), die abwechselnd mit dem Schubschild (4) entlang der Führungsschienen (11) im Abstand aufeinanderfolgender Förderschritte verriegelbar sind, und dem Schubschild (4) ein Vorschubantrieb (20) vorgesehen ist.

2. Vorrichtung nach Anspruch 1, **dadurch gekennzeichnet, daß** das Schubschild (4) zwischen auf den Führungswagen (9) gelagerten Längsschlitten (13) um eine Querachse (15) schwenkbar gelagert ist.

3. Vorrichtung nach Anspruch 2, **dadurch gekennzeichnet, daß** das Schubschild (4) um eine bodennahe Querachse (15) abschwenkbar zwischen den Längsschlitten (13) gelagert ist und eine befahrbare Brücke bildet.

4. Vorrichtung nach Anspruch 2 oder 3, **dadurch gekennzeichnet, daß** die Führungsschienen (11) im Abstand der Förderschritte Rastaufnahmen (23) für in den Führungswagen (9) und den Längsschlitten (13) gelagerte, an Stelltrieben (24) angeschlossene Verriegelungsbolzen (21, 22) aufweisen.
